# EUROPEAN PATENT APPLICATION

(11) **EP 1 095 948 A1**
(43) Date of publication of application: **02.05.2001**
(21) Application number: 99120899.2
(22) Date of filing: 28.10.1999
(51) Int. Cl.: C07K 16/00, C12N 15/10, C12N 15/13, C12Q 1/68, G01N 33/53, A61K 39/395, C12N 5/08, C12N 5/06, C12N 1/21

(54) **Idiotype vaccines**

(71) Applicant: Universitätsklinikum Freiburg, 79106 Freiburg (DE)
(72) Inventor: Veelken, Hendrik, 79104 Freiburg (DE); Osterroth, Frank, 79106 Freiburg (DE)
(74) Representative: Keller, Günter, Dr.

(57) **Abstract**

The present invention relates to methods for isolating DNA encoding immunoglobulin V-regions, methods for determining the idiotype of B cell lymphomas, methods for the preparation of idiotypic Fab fragments, Fab fragments produced by such methods and vaccines containing such Fabs. The invention also concerns methods for the stimulation of T cells. The invention further concerns the use of anchored PCR in those methods.

## Description

The present invention relates to methods for isolating DNA encoding immunoglobulin V-regions, methods for determining the idiotype of B cell lymphomas, methods for the preparation of idiotypic Fab fragments, Fab fragments produced by such methods and vaccines containing such Fabs.

The vast majority of malignant lymphoma cells have undergone rearrangement of antigen receptor genes and express antigen receptor molecules intracellularly or on their surface. The antigens that are recognized by the immunoglobulin receptors of B cell lymphomas have been identified in some cases and appear to be autoantigens. However, a systematic analysis of the antigen receptors has been limited by difficulties in obtaining lymphoma-derived immunoglobulin molecules that are correctly folded for antigen recognition.

In addition, the hypervariable regions of the immunoglobulins expressed by lymphoma cells (idiotypes) represent lymphoma-specific tumor antigens that may be target structures for anti-lymphoma immune responses themselves. Purified or recombinant lymphoma-derived immunoglobulins may be formulated into a tumor-specific vaccination therapy to elicit a specific host immune response. Treatment of lymphoma patients with idiotype protein has already been shown to be successful (Hsu et al. (1997) Blood 89, 3129; Bendandi et al. (1999) Nat. Med. 5, 1171).

In plasmacytoma and many cases of lymphoplasmacytoid immunocytoma, secreted idiotype para-protein can be purified conveniently from the blood of patients. In the case of non-secreting lymphomas, rescue of lymphoma cells by somatic cell hybridization with a cultured hybrid mouse-human lymphoma cell line permits production of lymphoma immunoglobulin in vitro (Caroll et al. (1986) J. Immunol. Methods 89, 61). However, this approach requires viable and sterile lymphoma cells as well as laborious and time-consuming cell culture techniques. To circumvent these limitations, molecular cloning of the immunoglobulin genes by PCR amplification with variable region family-specific primers, followed by construction of individual expression vectors for single-chain Fv (scFv) fragments of the respective idiotype has been proposed for DNA vaccination (Hawkins et al. (1994) Blood 83, 3279). To obtain recombinant lymphoma-derived scFv proteins for vaccination purposes, an expression strategy in tobacco plants was described recently (McCormick et al. (1999) Proc. Natl. Acad. Sci. USA 96, 703).

It is an object of the present invention to provide a method for determining the idiotype expressed by certain cells, e.g. B cell lymphoma cells, to allow rapid recombinant expression of idiotypic Fab fragments.

The present invention relates to a method for the preparation of DNA encoding at least the variable region of the immunoglobulin heavy chain or/and of the immunoglobulin light chain expressed by certain cells such as the B cells of a clinical sample. According to the method mRNA is isolated from the cells and cDNA is prepared from the mRNA by reverse transcription. An anchor sequence is then added to the 3' end of the cDNA. The anchor sequence consists of at least five nucleotides, preferably of at least ten nucleotides, more preferably of at least 20 nucleotides. The nucleotides of the anchor sequence may be added sequentially by using the enzyme terminal deoxynucleotidyl transferase which adds deoxynucleotides to the 3' end of DNA in the presence of deoxynucleoside triphosphates. For example, by adding dGTP and terminal transferase to the synthesized cDNA, an oligo-dG tail is added to the 3' end of the cDNA molecule. It is also possible to link oligonucleotides to the cDNA by ligation. Such oligonucleotides may be prepared by chemical synthesis. The resulting cDNA is amplified by polymerase chain reaction (PCR), wherein primer 1 (the 5' primer) hybridizes to the anchor sequence and primer 2 (the 3' primer) hybridizes to a sequence complementary to a sequence within the constant region of immunoglobulin heavy chain cDNA or of immunoglobulin light chain cDNA. For example, if an oligo-dG tail has been added as an anchor sequence to the 3' end of the cDNA, one primer of the PCR will comprise an oligo-dC stretch which is capable of hybridizing to the oligo-dG tail of the cDNA. The 5' primer may also comprise a sequence which can serve as a new anchor sequence for a second PCR. The primer corresponding to the constant region of the immunoglobulin gene may be directed to the constant region of any heavy chain gene or light chain gene. Depending on the immunoglobulin isotype different heavy chain genes are expressed. These heavy chain genes include the µ, α₁, α₂, δ, ε, γ₁, γ₂, γ₃, γ₄ gene. The constant region primers are directed against one of these genes. Therefore, it may be necessary to first determine the immunoglobulin isotype by other methods, for example by FACS. This is, however, usually determined during routine diagnostic immunophenotyping as described in Knowles D. M. (1993) Sem. Oncol. 20, 583. As to the light chains, there exist two types of light chain genes, the κ and the λ gene. Therefore, for amplifying the light chain gene, a κ or λ primer is used.

It is also possible to use a mix of constant region primers without prior determination of the immunoglobulin isotype. Thus, no time is lost due to negative PCR results.

90% of human B cell lymphomas express the IgM isotype, and two thirds express κ light chains. Therefore, when the cells are derived from a human B cell lymphoma, the primer directed against the constant region of an immunoglobulin heavy chain gene preferably hybridizes to the constant region of the µ-chain, and the primer directed against the constant region of the immunoglobulin light chain gene preferably hybridizes to the constant region of the κ gene.

In many cases a single PCR will not be sufficient to obtain enough DNA for cloning or sequencing. Therefore, a second PCR may be performed using nested primers. One primer of the second PCR (the 5' primer) hybridizes to a new anchor sequence introduced by the first PCR. The second primer of the second PCR (the 3' primer) is nested, i.e. it is directed to a C region sequence closer to the variable region of the respective chain than the 3' primer of the first PCR. The PCR product may be further purified by gel electrophoresis and subsequent isolation from the gel, for example by adsorption to a silica matrix. In order to enhance the sensitivity of the method, a third round of PCR may be performed using the anchor primer from the second PCR and a further nested primer for the constant region. Sensitivity can be further enhanced by extending the third round PCR reaction (using the same primers). Thereby, it is possible to obtain the desired DNA starting with RNA from only ten cells.

It is clear to the person skilled in the art that the methods described above can also be applied to T cell receptors (TCRs). More generally, the methods of the present invention described for immunoglobulin molecules relate to methods for antigen receptor molecules. Within the meaning of this application, antigen receptor molecules are either immunoglobulins or T cell receptors. T cell receptors are heterooligomers of 2 polypeptide chains. Similar to the heavy or light chains of immunoglobulins, polypeptide chains of TCRs comprise a constant region and a variable region. The mechanism for the generation of diversity is also similar. Polypeptides chains known to be part of TCRs are the α, β, γ, and δ chain. Examples of TCRs are αβ and γδ heterodimers.

The cells to be used as starting material are preferably human tumor cells, more preferably the cells are derived from a patient suffering from B cell lymphoma. Different forms of lymphoma are follicular lymphoma (FL), lymphocytic lymphoma or chronic lymphopathic leukemia (CLL), mantle cell lymphoma (MCL), hair cell leukemia (HCL), lymphoplasmacytoid immunocytoma (LPL), marginal zone lymphoma (MZL), multiple myeloma (MM), large cell lymphoma (LCL), and Burkitts lymphoma(BL). The cells may also be derived from a patient with T cell lymphoma. The clinical biopsies that can be used include lymph node biopsies and fine needle aspirations, ascites and pleural fluid, bone marrow biopsies, endoscopic biopsies of the gastrointestinal and bronchial tract, and stereotactic biopsies of the brain.

The nucleotide sequence of the amplified PCR product can be determined after cloning into an appropriate vector. Inspection of the nucleotide sequences of the variable regions of several clones reveals whether one V region sequence is predominant. For example, sequencing of nine clones derived from human lymphoma cells showed that seven clones were identical. The two different clones were derived from other lymphocytes present in the biopsy. The predominance of one V region sequence is a strong indication that the sequence represents the idiotype of the lymphoma.

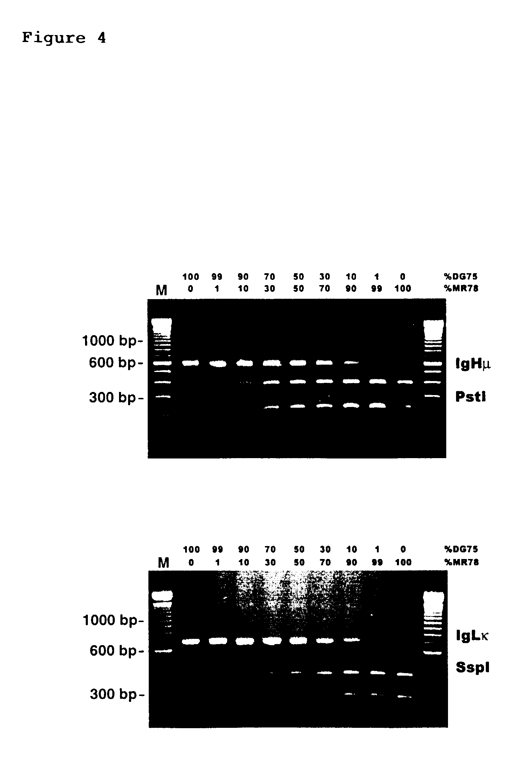

Another aspect of the invention is amplification of immunoglobulin transcripts in true proportion to the composition of the cells in the starting material (see Figure 4). Since the primer at the 3' terminus of the cDNA hybridizes to the anchor and is identical for all transcripts present in a PCR reaction, no amplification bias for certain sequences can occur. This aspect of the invention ensures that the lymphoma-derived immunoglobulin sequence is not underamplified or lost during the amplification process. This is important since when taken from a biopsy the cellular starting material always contains a mixture of different immunoglobulin transcripts even if the biopsy is taken from a lymphoma. Methods known in the art for amplifying variable region sequences often use forward primers that hybridize in the leader or framework 1 region of the V region. Such primers amplify only members of a certain family of V region sequences because of sequence variation in this region. This may lead to non-detectability of certain immunoglobulin sequences. The methods of the invention overcome this problem.

Another aspect of the invention is a method for the preparation of an Fab fragment of an immunoglobulin expressed by certain cells, e.g. by the B cells of the cellular starting material. DNA encoding a heavy chain and DNA encoding a light chain is isolated according to the method of the present invention. The DNA is subsequently cloned into an expression vector, host cells are transformed with the expression construct and eventually cultured under conditions such that the immunoglobulin heavy chain and immunoglobulin light chain are expressed.

Preferably, the host cells are bacterial cells, more preferably the host cells are E. coli cells.

To ensure stoichiometric expression of the heavy chain and the light chain, DNA is preferably expressed in a dicistronic manner. It is also preferred to include sequences into the expression construct that result in the secretion of the expression products into the periplasmic space of the bacteria. Examples are the leader peptide sequence of outer membrane protein (ompA) or of alkaline phophatase (phoA). The conditions in the periplasmic space allow correct folding of the immunoglobulin chains and assembly of heavy and light chains.

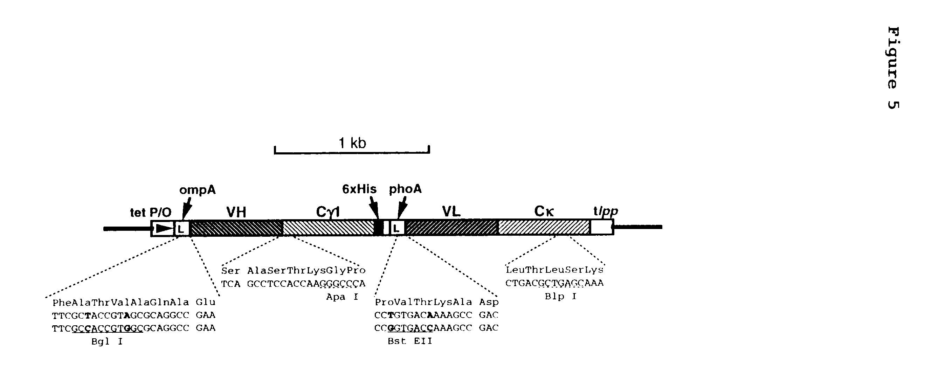

Usually a lymphoma biopsy from a patient is used as starting material. The immunoglobulin isotype of the lymphoma is determined by routine immunophenotyping. RNA is isolated from the cells followed by reverse transcription and amplification of the V region DNA sequence by anchored PCR (A-PCR). The sequences encoding the heavy chain and the light chain V regions are cloned into an appropriate vector and sequenced. Then the amplified heavy chain and light chain variable regions are cloned into a suitable expression vector such as pFab.yK (Figure 5). The vector contains the constant region sequence of the γ1 heavy chain and the constant region sequence of the κ light chain. The resulting construct is transformed into E.coli cells and fermented under GMP criteria. The periplasmic cell fraction is isolated and the respective individual Fab fragments are purified by known methods such as affinity chromatography. Preferably the Fab fragments are purified via a tag sequence comprising several histidine residues. Finally the quality of the resulting purified Fab fragment is controlled with respect to purity, endotoxin content and sterility.

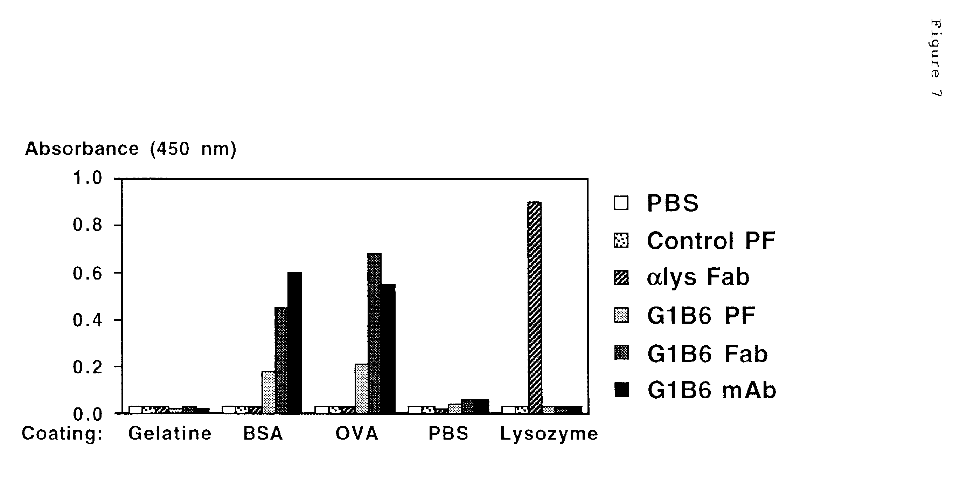

Another aspect of the present invention are Fab fragments prepared by a method according to the present invention. In particular, the invention relates to a Fab fragment produced by recombinant techniques containing no foreign amino acids within the immunoglobulin sequences. This is especially important when the recombinant Fab fragments are to be used as vaccines. If foreign amino acids are present, there is the risk that novel antigenic sites may be generated. If a histidine tag is present in the Fab fragment it is preferably at the C terminus of the polypeptide chain. Up to date no T cell responses have been observed against His₆ tags. Therefore, presence of a histidine tag does not contradict the use of the Fab fragment as a vaccine. Antigen binding of the Fab fragment is also not influenced by a histidine tag (see Figure 7). When no tag is present, purification can be achieved by affinity chromatography using a protein G column or an anti-immunoglobulin column.

Fragments of TCRs can also be expressed by recombinant methods. Since recombinant TCR molecules often are insoluble purification under denaturing conditions is possible.

The invention also relates to a vaccine comprising an Fab fragment according to the present invention. If an Fab fragment is to be used for vaccination then in 5% of the total amount of the preparation to be applied bacteria or fungi must not be detected. Furthermore, the endotoxin content must be below 10 U/mg protein and the Fab fragment has to be at least 90% of the total protein content as determined by SDS-PAGE. The recombinant Fab fragment solution is preferably adjusted to a protein content of 20 mg/ml and mixed with the same volume of the lipid based adjuvans MF-59. MF-59 can be obtained in GMP quality. Repeated vaccinations may be performed with doses that vary from about 0,5 mg to 5 mg per vaccination. The application of the vaccine is preferably intracutaneously or subcutaneously, but other routes of administration are possible.

TCR molecules or fragments thereof may also be used as vaccines against T cell lymphomas.

Another aspect of the invention is the property of the produced Fab fragments to bind antigen in a specific interaction. This property permits to identify the antigen recognized by the respective lymphoma cells by means of immunohistochemistry, flow cytometry, ELISA, Western blot analysis, or immunoprecipitation. This aspect may permit to gain important information about the origin and biological behaviour of B cell lymphoma cells.

Another aspect of the invention is a method for the preparation of stimulated cytotoxic T cells against idiotype immunoglobulin of malignant lymphoma (see example 6). According to the method, the idiotype of the lymphoma is determined by a method described in this application. Autologous antigen presenting cells (APC) are provided in in vitro culture. Preferably dendritic cells (DC) are used. DC can be generated from peripheral blood mononuclear cells (PBMC). The APC have to be "loaded" such that they present fragments of the idiotype immunoglobulin on their surface. This can be achieved by different methods. Idiotypic Fab fragments can be prepared from the DNA encoding the idiotype by a method according to the invention. The Fab fragments are then contacted with the APC in in vitro culture. After internalization by the APC the Fab fragments are processed intracellularly and eventually presented on the surface of the APC. An alternative way is to express in APC DNA sequences encoding idiotype protein. This can be achieved by using known viral or non-viral gene delivery methods such as transduction by semliki forest virus. Processing of exogenous idiotype Fab fragments is preferred according to the invention. The APC presenting fragments of the idiotype immunoglobulin on their surface are then contacted with autologous T cells under appropriate conditions (see example 6) such that cytotoxic T cells are stimulated. The stimulated T cells can be transferred back into the lymphoma patient to enhance the immune response against the tumor cells. In a similar manner, the invention can be used for specific immunotherapy of a lymphoma patient who has been treated with allogeneic stem cell transplantation. Cytotoxic T cells of the stem cell donor can be stimulated with APC cells from the same donor against the tumor-derived idiotype immunoglobulin of the lymphoma patient. The stimulated donor T cells can then be transferred into the patient to enhance the transplanted immune system of the donor against the tumor cells.

An advantage of the described methods is that idiotype-specific cytotoxic T cells can be prepared by a GMP-compatible protocol without the requirement of viable lymphoma cells.

T cells can also be stimulated using as an antigen TCR molecules or fragments thereof. Insolubility of recombinantly expressed TCR molecules is not a problem because the antigens are usually denatured before loading the APC with antigen. Therefore, TCR molecules or fragments thereof may well be used for vaccination against T cell lymphomas such as mycosis fungoides.

Another aspect of the invention is a stimulated cytotoxic T cell produced by the described method of the invention.

The invention further concerns the use of anchored PCR for the isolation of immunoglobulin genes, for determining the immunoglobulin idiotype, for the preparation of recombinant Fab fragments, and for the preparation of stimulated cytotoxic T cells.

The present invention provides methods for the preparation of Fab fragments that are highly useful as individual idiotype vaccines for patients with malignant lymphoma. Currently available techniques employ somatic cell hybridization or expression in tabacco plants. The methods of the present invention have many advantages when compared with those techniques: The preparation takes only three weeks versus six months (prior art techniques). The prior art techniques are much more laborious (animal or plant cell culture versus bacterial cell culture). Purification of cell culture supernatant is much more expensive than the methods of the invention. Finally, the invention provides an exactly defined molecular system with high reliability. This is important with respect to national health regulations.

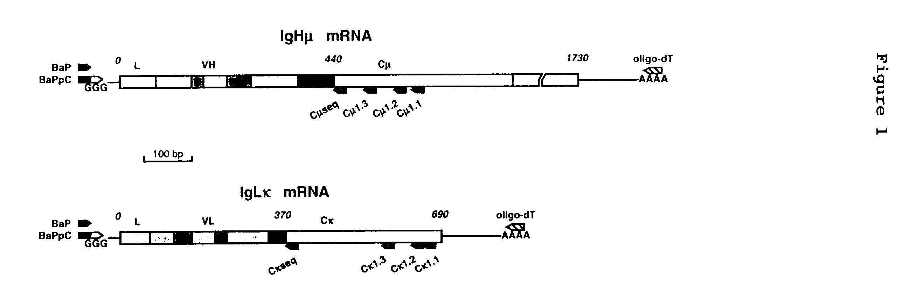

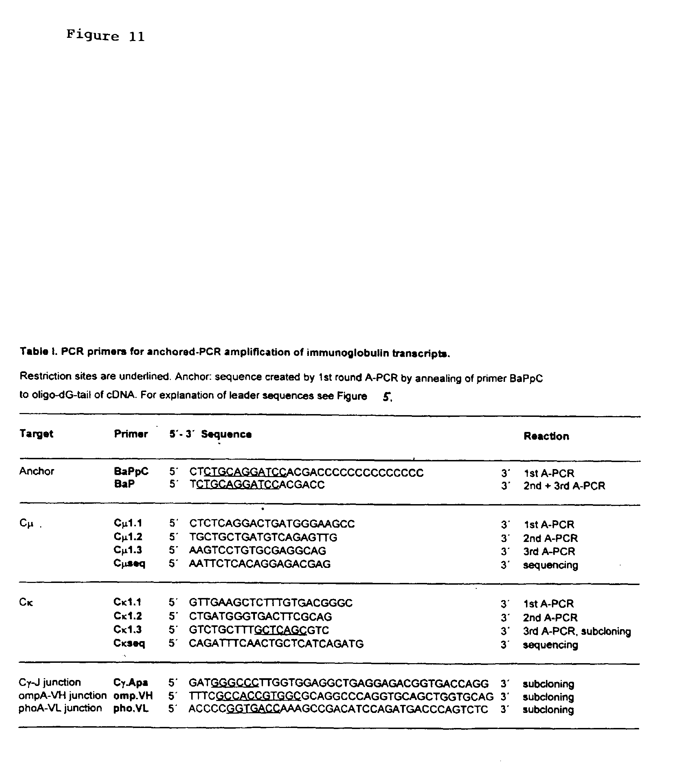

Figure 1 shows the schematic representation of preferred primer positions for reverse transcription, anchored-PCR amplification, and sequencing of IgHµ and IgLκ transcripts. The primer sequences are described in more detail in figure 11 (table 1). Table 1 shows preferred PCR primers for anchored-PCR amplification of immunoglobulin transcripts.
Primer BaPpC corresponds to SEQ ID NO: 1.
Primer BaP corresponds to SEQ ID NO: 2.
Primer Cµ1.1 corresponds to SEQ ID NO: 3.
Primer Cµ1.2 corresponds to SEQ ID NO: 4.
Primer Cµ1.3 corresponds to SEQ ID NO: 5.
Primer Cµseq corresponds to SEQ ID NO: 6.
Primer Cκ1.1 corresponds to SEQ ID NO: 7.
Primer Cκ1.2 corresponds to SEQ ID NO: 8.
Primer Cκ1.3 corresponds to SEQ ID NO: 9.
Primer Cκseq corresponds to SEQ ID NO: 10.
Primer Cγ.Apa corresponds to SEQ ID NO: 11.
Primer omp.VH corresponds to SEQ ID NO: 12.
Primer pho.VL corresponds to SEQ ID NO: 13.
The use of the primers is described in the examples.

Figure 2 shows amplified IgHµ and IgLκ transcripts from 3 B cell lines and 9 clinical biopsies after A-PCR amplification. M = size marker (100 bp ladder, Gibco BRL).

Figure 3 shows amplified IgHµ and IgLκ transcripts from the cell line DG75. On top of each column the number of cells is indicated that were used as starting material for the preparation of RNA. M = size marker (100 bp ladder, Gibco BRL).

Figure 4 shows the proportional representation of different IgHµ and IgLκ transcripts in A-PCR products obtained from mixtures of the B cell lines DG75 and MR78. M = size marker (100 bp ladder, Gibco BRL).

Figure 5 shows a schematic map of the prokaryotic Fab fragment expression plasmid pFab.γκ.

tet P/O: tet promoter/operator element. ompA: leader peptide sequence of outer membrane protein A. phoA: leader peptide sequence of alkaline phosphatase. tipp: lipoprotein transcription terminator. 6xHis: string of 6 histidine codons. The sequences below the map encompass the recognition sites (underlined) for insertion of PCR-amplified variable gene segments. Conservative base changes introduced to create artificial restriction sites are depicted in bold letters (upper sequence: pASK88, lower sequence pFab.γκ). Apart from the new restriction sites, the vector corresponds to pASK88.

The specific sequences shown are: Peptide sequence at the ompA-VH junction SEQ ID NO: 14; DNA sequence at the ompH-VH junction of pASK88: SEQ ID NO: 15; DNA sequence at the ompA-VH junction of pFab.γK: SEQ ID NO: 16; Peptide sequence at the VH-Cγ1 junction: SEQ ID NO: 17; DNA sequence at the VH-Cγ1 junction: SEQ ID NO: 18; Peptide sequence at the phoA-VL junction: SEQ ID NO: 19; DNA sequence at the phoA-VL junction of pASK88: SEQ ID NO: 20; DNA sequence at the phoA-VL junction of pFab.γκ: SEQ ID NO: 21; Peptide sequence from the CK region: SEQ ID NO: 22; DNA sequence from the Cκ region: SEQ ID NO: 23.

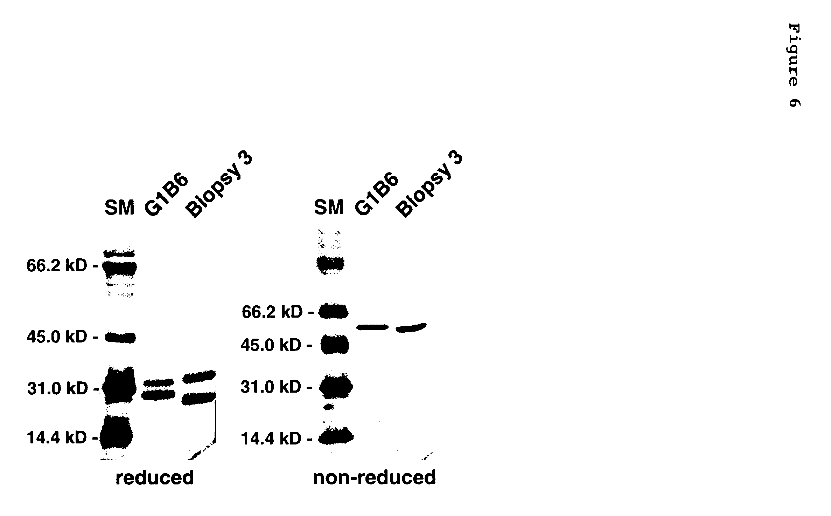

Figure 6 shows a silver stained SDS-polyacrylamide gel after separation of recombinant, affinity-purified Fab fragments. SM = high molecular weight marker (Gibco).

Figure 7 shows the result of an ELISA to determine the antigen specificity of a recombinant Fab fragment.

BSA: bovine serum albumin. OVA: ovalbumin. Control PF: periplasmatic fraction of untransformed bacteria. αlys Fab: purified recombinant anti-lysozyme Fab fragment. G1B6 PF: periplasmatic fraction with recombinant G1B6 Fab fragment. G1B6 Fab: affinity chromatography-purified recombinant G1B6 Fab fragment. G1B6 mAb: tissue culture supernatant of G1B6 hybridoma cell line.

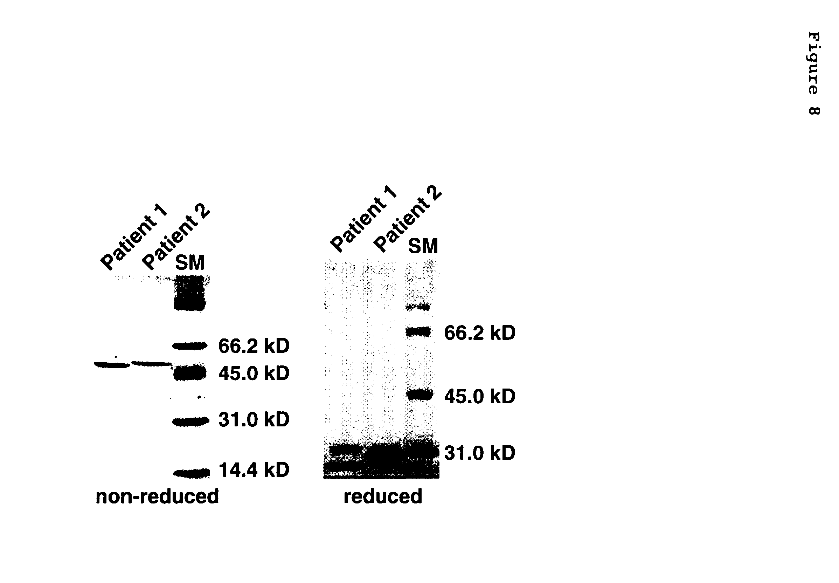

Figure 8 shows an SDS-PAGE analysis of recombinant, lymphoma-derived Fab fragments. Immunglobulin transcripts from lymphoma biopsies of both patients were amplified by A-PCR, sequenced, and inserted into pFab.γκ. Recombinant Fab fragments were expressed from pFab.γκ vectors in E. coli. Periplasmic fractions of induced bacteria were prepared and purified by affinity chroatography. The resulting protein eluate was analyzed by standard 12% SDS-polyacrylamide gel electrophoresis under reducing and non-reducing conditions. Protein bands were visualized by silver staining. The Fd and IgL chains of patient 2 has almost identical migration properties under reducing conditions. Even when the gel was run with 6 M urea, the separation of the bands could not be improved. SM: high-molecular weight marker (Gibco).

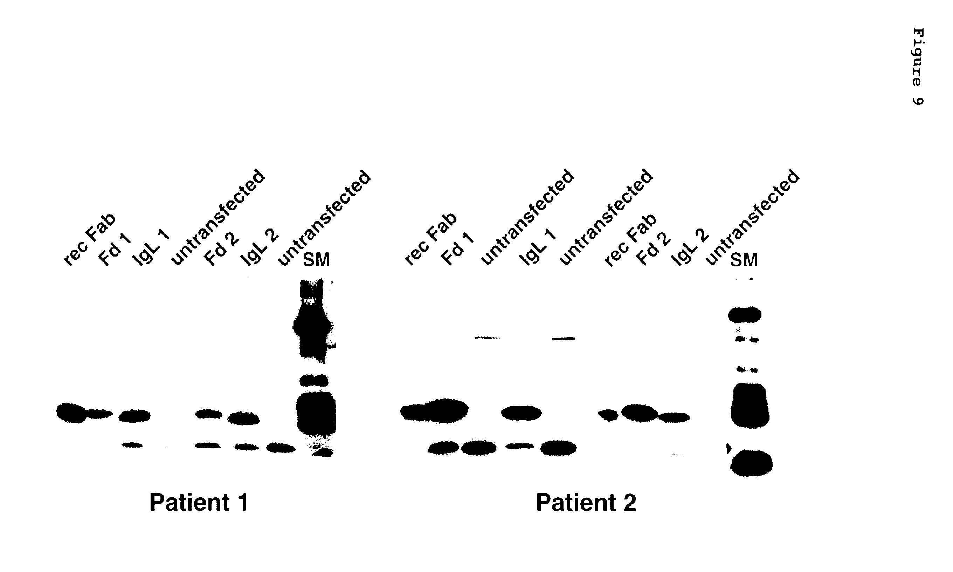

Figure 9 shows expression of recombinant idiotype in stably transfected LCL. Lymphoma-derived immunglobulin genes were inserted with an attached hexahistidine tag into the EBV-replicon vector pCEP4. Well-proliferating LCL were electroporated and selected with hygromycin. Hygromycin-resistant LCL were lysed and analyzed by Western blot with an anti-histidine antibody. Bound antibody was detected by chemiluniniscence. rec Fab: Recombinant Fab fragment loaded as control. Fd1, IgL1: LCL transfected with pCEP4 vectors encoding the heavy and light chain idiotype gene of patient 1, respectively. Fd2, IgL2: LCL transfected with pCEP4 vectors encoding the heavy and light chain idiotype gene of patient 2, respectively. SM: 10-250 kD molecular weight marker (Amersham).

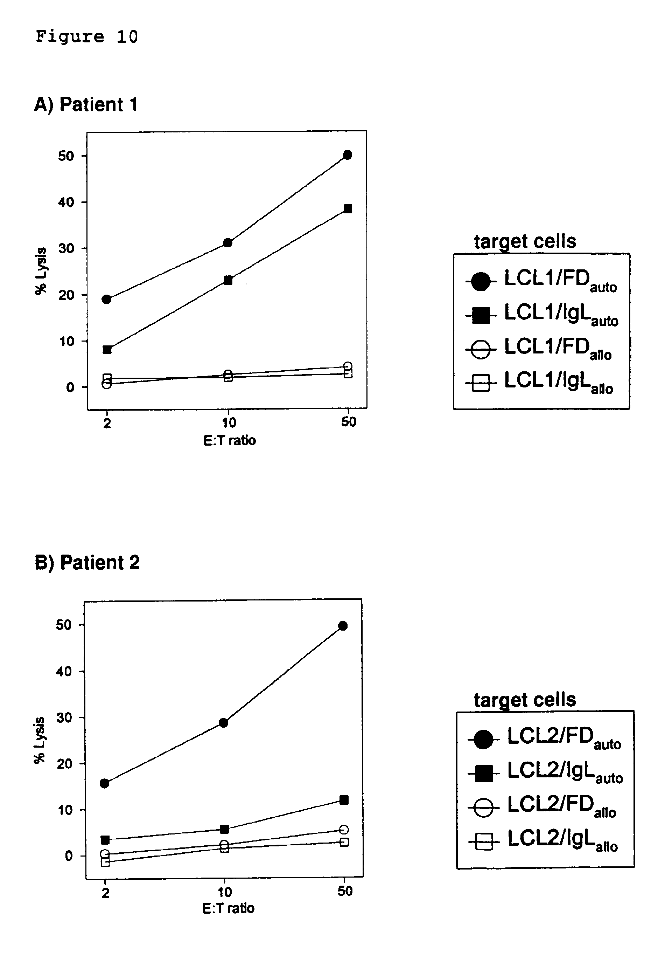

Figure 10 shows an analysis of the cytotoxic activity of the stimulation experiments of patient 1 and 2 using autologous T cells. The stimulation of CD8⁺ T lymphocytes was performed using Fab loaded dendritic cells.
*A) Patient 1*: The cytotoxicity was analyzed one week after the third restimulation by a standard ⁵¹Cr release assay using the transfected lymphoblastoid cell lines of patient 1 (LCL1). The target cell lines for the detection of the specific lytic activity expressed either the idiotypic light chain (LCL1/IgLₐᵤₜₒ or the idiotypic F_{D} chain (LCL1/FDₐᵤₜₒ). The lymphoblastoid cell lines transfected with the corresponding expression vector of patient 2 served as a control target (LCL1/IgLₐₗₗₒ or LCL1/FDₐₗₗₒ).
*B) Patient 2*: The cytotoxicity was analyzed one week after the third restimulation by a standard ⁵¹Cr release assay using the transfected lymphoblastoid cell lines of patient 2 (LCL2). The target cell lines for the detection of the specific lytic activity expressed either the idiotypic light chain (LCL2/IgLₐᵤₜₒ or the idiotypic F_{D} chain (LCL2/FDₐᵤₜₒ). The lymphoplastoid cell lines transfected with the corresponding expression vector of patient 1 served as a control target (LCL2/IgLₐₗₗₒ or LCL2/FDₐₗₗₒ).

The following examples illustrate the invention.

### Example 1

### Anchored PCR-mediated cloning of Ig heavy chain (IgH) and Ig light chain (IgL) genes.

Total RNA was isolated from clinical biopsies or cultured cell lines by centrifugation-driven chromatography through a silica matrix under conditions given by the manufacturer (RNeasy columns, Qiagen, Hilden, Germany). Oligo-dT-primed cDNA was synthesized from approximately 6 µg of RNA with RNaseH-deficient reverse transcriptase (Gibco BRL). A selection for cDNA of more than 400 bp was performed by spun-driven chromatography through Sepharose CL-4B (Pharmacia, Uppsala, Sweden). An oligo-dG tail was added to size-selected cDNA with terminal transferase (Boehringer Mannheim, Mannheim, Germany) in the presence of 0.25 mM dGTP under reaction conditions given by the manufacturer. This material was subjected to a first round of PCR amplification with recombinant Taq polymerase (Perkin Elmer, Norwalk, CT). The first round of A-PCR was performed for 20 cycles with an annealing temperature of 60°C and primer Cµ1.1 (SEQ ID NO: 3) for amplification if IgH cDNA and primer Cκ1.1 (SEQ ID NO: 7) for IgL cDNA, respectively, in conjunction with primer BaPpC (SEQ ID NO:1). After gel electrophoresis of the products, gel slices corresponding to the bands or to the anticipated position of full-length PCR products were excised from the gel. The gel slices were squeezed, and the eluate was reamplified for 25 cycles with primers Cµ1.2 (SEQ ID NO: 4) or Cκ1.2 (SEQ ID NO: 8) and primer BaP (SEQ ID NO: 2) at an annealing temperature of 57°C. To increase the overall yield and specificity, a third-round PCR was performed for 25 cycles with 1 µl of the second-round PCR product with nested primers Cµ1.3 (SEQ ID NO: 5) or Cκ1.3 (SEQ ID NO: 9) and primer BaP (SEQ ID NO: 2) under otherwise indentical conditions.
All PCR amplifications were performed with 25 pmol of each primer and 1.25 U of Taq polymerase (Perkin Elmer, Norwalk, CT) in a total volume of 50 µl. The final PCR buffer was 10 mM Tris-Cl (pH 8,3)/1.5 mM MgCl₂/50 mM KCl. A PCR cycle consisted of denaturation at 94°C for 1 min, annealing for 1 min (temperatures as given above), and extension at 72°C for 1 min in a DNA thermal cycler 480 (Perkin Elmer).

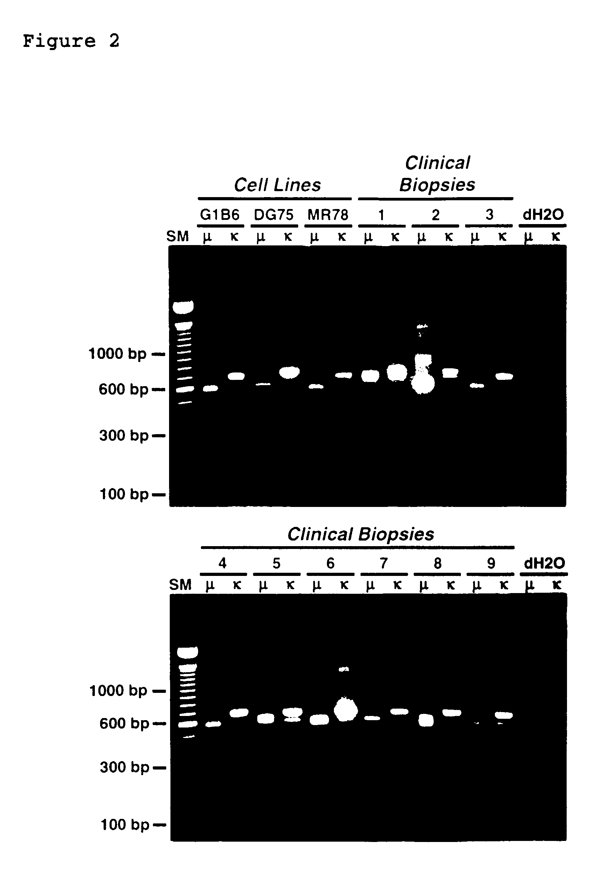

PCR products were analyzed by 1% agarose gel electrophoresis and staining with ethidium bromide. Intensely stained bands were reliably observed at the anticipated positions in agarose gels after the third round of PCR amplification of cDNA derived from cell lines and lymphoma biopsies. The results are shown in Figure 2.

### Example 2

### Sensitivity of the A-PCR technique for successful amplification of immunoglobulin transcripts from limited numbers of B cells.

Total RNA was extracted from decreasing numbers of DG75 cells and subjected to A-PCR amplification of IgHµ and IgLκ transcripts. Three rounds of A-PCR were performed according to Example 1.

10% of final PCR products were electrophoresed through 1% agarose and visualized by staining with ethidium bromide.

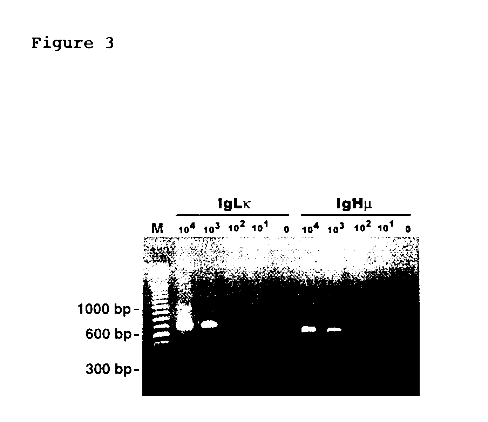

The result is shown in Figure 3.

### Example 3

### Proportional representation of different IgHµ and IgLκ transcripts in A-PCR products obtained from mixtures of two B cell lines.

DG75 and MR78 B lymphoma cells were mixed in various proportions as indicated in Figure 4. RNA from 10⁵ cells of each mixture was amplified by A-PCR for IgHµ and IgLκ transcripts. A-PCR products were digested with Pst I (IgH amplifications) or Ssp I (IgL amplifications), respectively. These restriction enzymes cut only once in the respective MR78-derived but not in the DG75-derived variable gene segments. The restriction digests were analyzed by 1% agarose gel electrophoresis and staining with ethidium bromide.

The result is shown in Figure 4.

### Example 4

### Functional expression of recombinant Fab fragments.

The dicistronic prokaryotic expression vector pASK88 was designed for the bacterial secretion of functional Fab fragments carrying human Cκ and Cγ1 domains as well as a histidine (His₆) tag (Skerra, 1994a; Skerra, 1994b; Schiweck et al., 1997). To avoid the presence of non-natural codons within the Fab genes, several conservative base changes were introduced by PCR-mediated mutagenesis to create unique Bgl I and Bst EII restriction sites within the bacterial signal peptides of pASK88 (Figure 5). An additional Bgl I restriction site located within the bacterial amp gene of pASK88 was eliminated by an artificial silent point mutation. Together with unique Apa I and Blp I sites in the human Cγ and Cκ segments, these modifications permit directional insertion of tumor-derived variable gene segments without creating non-natural peptide sequences. When recombinant Fab fragments are expressed from the resulting vector pFab.γκ (Figure 5), the His₆ tag at the C-terminus of the heavy chain constitutes the only remaining artificially introduced amino acid residues after cleavage of the leader sequences.

Cloned, G1B6-derived IgHµ and IgLκ variable gene sequences were reamplified for 20 cycles with an annealing temperature of 57°C with primer pairs Cγ.Apa (SEQ ID NO: 11) - omp.VH (SEQ ID NO: 12) and Cκ1.3 (SEQ ID NO: 9) - pho.VL (SEQ ID NO: 13), respectively (Table I). These primers carry the restriction sites necessary for directional subcloning of the PCR products into pFab.γκ. Insertion of the PCR products into pFab.γκ resulted in the expression vector pFab.G1B6.

After assembly of the expression vector, E. coli JM83 were transformed, grown to an OD₅₅₀ of 0.5 in LB medium, and induced with 0.2 mg/l anhydrotetracyclin (Fisher Scientific, Nidderau, Germany) for 3 h. To prepare the periplasmic cell fraction, pelleted bacteria were lyzed with 100 mM Tris-HCl (pH 8.0)/0.5 M sucrose/1 mM EDTA on ice for 30 min. Spheroplasts were separated from the lysate by centrifugation for 10 min at 4000 g. After a second centrifugation for 15 min at 27000 g, the supernatant was dialyzed over night against 0.5 M betain monohydrate (Fluka, Deisenhofen, Germany)/50 mM Na phosphate, pH 7.5. Recombinant Fab fragments were purified from these periplasmatic cell fractions by affinity chromatography on a Zn²⁺ column (chelating sepharose fast flow, Pharmacia) as described. Purified recombinant Fab fragments were analyzed by standard 12% SDS-PAGE electrophoresis under reducing and non-reducing conditions. SDS-PAGE of the purified protein demonstrated a virtually pure band of the expected size for a Fab heterodimer after silver staining, and SDS-PAGE analysis under reducing conditions revealed bands of the expected size for monomeric IgH Fd fragments and IgL chains (Figure 6).

Recombinant Fab fragments obtained from clinical biopsies were of similar purity as the G1B6 Fab fragment as shown for biopsy 3, a lymph node with infiltration of a diffuse large cell lymphoma (Figure 6). The overall yield of recombinant Fab fragments was 220-250 µg per L of bacterial cell culture. Residual endotoxin content was 0.115 EU (G1B6) and 0.059 EU (biopsy 3) per µg of recombinant Fab fragment.

### Example 5

### Antigen recognition of a recombinant Fab fragment.

To determine whether the recombinant, purified Fab fragments had indeed the correct conformation for antigen binding, an ELISA assay was established to compare binding of the G1B6-derived Fab fragment with the native monoclonal antibody from cell culture supernatant. The antibody G1B6 recognizes various albumins, but not collagen or gelatine. 96-well microtiterplates (Sigma, Deisenhofen, Germany) were coated with 40 mg/l ovalbumin (grade V, Sigma), 40 mg/l bovine serum albumin (Sigma), or 40 mg/l lysozyme (Biomol, Hamburg, Germany). After two washes with PBS, the plates were saturated with 15 g/l gelatine (Sigma). Recombinant Fab fragments or hybridoma culture supernatant were added and incubated for 2 h at room temperature (RT), followed by four washes with PBS. Bound antibody was detected by incubation with a goat-anti-human kappa-alkaline phosphatase conjugate (Biosource Europe, Fleurus, Belgium) at a dilution of 1:10,000 for 2 h, followed by four additional washes. Finally, 200 µl p-nitrophenyl phosphate liquid substrate (Sigma) was added. After 30 min incubation at RT, the signal was measured at 405 nm with an automated ELISA reader (DPC, Los Angeles, CA). Endotoxin contamination of purified recombinant proteins was measured with a limulus amebocyte assay (BioWhittaker, Walkersville, MD). Binding of the monoclonal G1B6 antibody, an unpurified periplasmic cell fraction from pFab.G1B6-transformed bacteria, and the purified recombinant Fab fragment was detected with a secondary goat-anti-human antibody conjugated with alkaline phosphatase. The result is shown in Figure 7. As indicated by this qualitative ELISA assay, the recombinant Fab fragment is able to specifically bind antigen in a similar manner to its corresponding, native monoclonal antibody.

### Example 6

### Stimulation of cytotoxic T cells against idiotype immunoglobulin of malignant lymphoma with protein-pulsed dendritic cells

### a) Patient samples

Biopsy specimen with histologically and immunophenotypically confirmed infiltrations of malignant lymphoma cells were obtained as non-sterile, single cell suspensions from the diagnostic immunophenotyping laboratory of the university hospital of Freiburg and stored at -70°C. The respective patients gave informed consent for use of their cryopreserved tumor cells and for acquisition of additional heparinized blood samples from routinely performed phlebotomies during complete clinical remission. The experiments were approved by the local ethics committee.

Patient 1 was a 43 year-old male diagnosed with a non-secreting lymphoplasmacytoid immunocytoma, stage IIIB. The surface immunoglobulin of the tumor cells was IgMκ. The patient was treated successfully by polychemotherapy, including high-dose BEAM chemotherapy with autologous peripheral stem cell transplantation. The patient was in clinical complete remission for 11 months when the first peripheral blood samples were taken.

Patient 2 was a 57 year-old female suffering from a IgMκ-positive follicular lymphoma, stage IIA, with involvement of cervical and submandibular lymph nodes. She received extended-field radiotherapy (40 Gy) with curative intent and was in complete remission for 6 months when the first blood samples were obtained.

### b) Production of lymphoma-derived idiotype protein as recombinant Fab fragment

RNA isolation, reverse transcription, cloning of the variable region sequences and expression of Fab fragments was performed as described in examples 1 and 4. The purity and integrity of the Fab fragments was assessed by standard 12% SDS-PAGE analysis and silver staining as shown in Figure 8. Endotoxin contamination of purified recombinant proteins was measured with a limulus amebocyte assay (BioWhittaker, Walkersville, MD).

### c) Generation of human monocyte-derived dendritic cells (DC)

Fresh peripheral blood mononuclear cells were prepared by Ficoll density centrifugation, suspended in RPMI/0.5% human serum albumin (HSA fraction V; Calbiochem, La Jolla, USA) and allowed to adhere to tissue culture flasks for 2 h. Non-adherent cells were removed by 3× washing with PBS. Adherent cells were differentiated into Langerhans-type cells (LC) in serum-free monocyte medium (Cellgenix, Freiburg, Germany) supplemented with 100 ng/ml GM-CSF (Novartis), 50 ng/ml IL-4, and 5 ng/ml TGF-β1 (both Genzyme, Cambridge, USA) for six days. Final differentiation to mature DC was induced by addition of 100 ng/ml lipopolysaccharide (Pyroquant Diagnostik, Walldorf, Germany). 24 h later, the cell population was used for stimulation of CTL (see below). Flow cytometry analysis of LC and DC was performed with antibodies specific for CD1a, CD14, and CD86 (Becton-Dickinson, Mountain View, USA).

### d) Generation and analysis of protein-loaded DC

For loading of DC with idiotype protein, 1 mg/ml solutions of recombinant Fab fragment were denatured by heating to 80°C in a microwave oven. This material was added to immature DC at a concentration of 50 µg/ml on day 5 of culture. To analyse the uptake of recombinant Fab fragments by immature DC, 10 µl of the fluorescent dye FLUOS (Boehringer Mannheim, Germany) were incubated with 660 µg of recombinant Fab fragment for 2 h at room temperature (RT). The protein was purified from unbound chromophore by gel filtration through Sephadex G-25 (Pharmacia). Photometrical analysis allowed calculation of the ratio of fluorescein molecules per protein by the formula 3.053 × OD₄₉₅ₙₘ/OD₂₈₀ₙₘ-0.255 × OD₄₉₅ₙₘ. In typical experiments, the FLUOS/protein ratio was approximately 15. 6 h after addition of FLUOS-labelled denatured Fab protein to LC, 10⁵ cells per spot were pipetted onto a polylysin-coated 12 spot glass slide and allowed to adhere for 30 min at 4°C. After fixation with 3% paraformaldehyde, cells were analyzed by confocal laser microscopy (LSM 410 Invert microscope, Zeiss, Oberkochen, Germany). In cells with DC morphology, bright fluorescence was present in cellular subcompartments with typical shape and localization of endosomes.

### e) Isolation of CD8⁺ PBMC and stimulation of CTL with DC

PBMC were isolated from heparinized blood samples by Ficoll density centrifugation. CD8⁺ cells were purified by depletion of CD4⁺, CD11b⁺, CD16⁺, CD19⁺, CD36⁺, and CD56⁺ cells with hapten-coupled antibodies against these antigens. Antibody-loaded cells were removed with a secondary hapten-specific antibody coupled to magnetic microbeads (MACS CD8⁺ T cell isolation kit, Miltenyi Biotech, Bergisch Gladbach, Germany). The culture medium for T cell stimulation consisted of RPMI supplemented with 2 mM L-glutamin, 0.55 mM L-arginin, 0.24 mM L-asparagin, 1 mM sodium pyruvate, MEM vitamins 1:200, MEM non-essential amino acids 1:100, 0.1 µM 2-mercaptoethanol, 50 U/ml penicillin, 50 µg/ml streptomycin (all Gibco BRL), and 5% (v/v) autologous human plasma. 5×10⁴ CD8⁺ PBMC were stimulated with 2×10⁴ mature DC in round-bottom microtiter wells in 210 µl medium supplemented with 1000 U/ml IL-6 and 10 ng/ml IL-12 (both Boehringer Mannheim). T cells were pooled and restimulated with DC at the same cell ratio in weekly intervals. After restimulations, cells were cultured in medium supplemented with 20 U/ml IL-2 and 5 ng/ml IL-7 (both Boehringer Mannheim). During the entire stimulation period, 70 µl of the respective culture medium was renewed every 2-4 days.

### f) Generation of idiotype-expressing lymphoblastoid cell lines (LCL)

These cells served as target cells to assess idiotype specific cytotoxicity. 10⁷ PBMC were resuspended in 2 ml EBV-containing supernatant. After an incubation for 2 h at 37°C, 4 ml RPMI 1640 supplemented with 10% fetal calf serum (PAA laboratories, Linz, Austria), 2 mM glutamin, 1 mM sodium pyruvate, MEM vitamins 1:200, MEM non-essential amino acids 1:100, 0.1 µM 2-mercaptoethanol, 50 U/ml penicillin, 50 µg/ml streptomycin (all Gibco BRL), and 1 µg/ml cyclosporin A (Novartis, Basel, Switzerland) were added. Fresh medium without cyclosporin was added once per week until outgrowth of EBV-transformed permanent cell lines occurred.
To obtain replicon-based idiotype expression vectors, individual clonally rearranged IgL genes and heavy chain Fd segments (i.e. the heavy chain variable region with the first constant domain) were amplified from the respective pFab.γκ clones with Vent polymerase. In addition to sequences complementary to the cDNAs, the 5' (forward) primers contained a SfiI restriction site, a Kozak consensus sequence, and an ATG start codon, whereas the 3' (reverse) primers incorporated the necessary codons for a hexahistidin tag, followed by two stop codons and a BamHI site. The PCR products were inserted into the SfiI/BamHI sites of pCEP4 (Invitrogen). 2×10⁷ vigorously proliferating LCL were electroporated with 20 µg pCEP4 vector with an electric discharge of 250 V and 960 µF in 0.4 cm cuvettes (Biorad, Hercules, USA). Successfully transfected cells were selected with 50 µg/ml hygromycin (Boehringer Mannheim) for one week and with 100 µg/ml thereafter.

### g) Western blot analysis for expression of recombinant idiotype

5×10⁷ pCEP4-transfected LCL were harvested and lysed in 100 µl 1% (v/v) nonidet P40 in 50 mM Tris-Cl (pH 7.6)/150 mM NaCl/2 mM EDTA. After a short centrifugation to separate particulate material, 15 µl of lysate was electrophoresed on a standard 12% SDS-polyacrylamide gel. Protein was transferred to a PVDF membrane (Hybond-P, Amersham-Buchler, Braunschweig, Germany) by electroblotting with 40 mA for 90 min (Transblot semidry, Biorad). The membrane was blocked with 5% milk powder in PBS/0.1% Tween 20 (blocking buffer) for 1 h at RT. The primary antibody (Invitrogen) diluted 1:2500 in blocking buffer. After 2 washes with 0.1% Tween 20 in PBS, the membrane was incubated with a sheep anti-mouse IgG-alkaline phosphatase conjugate (Amersham) 1:4000 in blocking buffer for 1 h. After three washes with 0.1% Tween 20 in PBS, specific binding was detected with the ECL Western blot detection system (Amersham) according to the instructions of the manufacturer. The result is shown in Figure 9.

### h) Detection of cytotoxic activity

10⁶ LCL were incubated with 10-40 µl Na₂⁵¹CrO₄ (Amersham) for 1 h at 37°C. After two washes with RPMI, 2000 target cells were plated per well into V-bottom microtiter plates. Effector cells were added at a effector-to-target ratio of 50:1, 10:1 and 2:1 in duplicates or triplicates. For maximal lysis, 0.15% Triton-X in medium was added. After a 4 h-incubation at 37°C, 50 µl of supernatant was counted in a scintillation counter (Lumaplate, Canberra-Packard, Dreieich, Germany). Specific lysis was calculated by the formula (specific release-spontaneous release)/(maximum release-spontaneous release). The experiments were performed as cold target inhibition assays with an excess of the typical natural killer target cell line K562 (10⁵ unlabelled K562 cells) in order to exclude natural killer activity.

The result of the cytotoxicity assay is shown in Figure 10.

## Claims

**1.** A method for the preparation of DNA encoding at least the variable region of a polypeptide chain of an antigen receptor from cells expressing the antigen receptor comprising
(a) providing mRNA from the cells
(b) preparing cDNA from the mRNA by reverse transcription
(c) adding an anchor sequence comprising at least 5 nucleotides to the 3' end of the cDNA
(d) amplifying the cDNA from (c) by polymerase chain reaction wherein primer 1 hybridizes to the anchor sequence and primer 2 hybridizes to a sequence complementary to a sequence within the constant region of the cDNA of the antigen receptor polypeptide chain.

**2.** A method according to claim 1, characterized in that after the polymerase chain reaction the DNA is further amplified by a second polymerase chain reaction wherein primer 1 of the second PCR hybridizes to a new anchor sequence introduced by the first PCR, and primer 2 of the second PCR hybridizes to a sequence complementary to a sequence within the constant region of the cDNA of the antigen receptor polypeptide chain which sequence is closer to the variable region than the sequence corresponding to primer 2 of the first PCR.

**3.** A method according to claim 1 or 2, characterized in that the anchor sequence is added by terminal deoxynucleotidyl transferase (terminal transferase).

**4.** A method according to any of claims 1 to 3, characterized in that the cells are tumor cells.

**5.** A method according to any of claims 1 to 4, characterized in that the cells are human lymphoma cells.

**6.** A method according to any of claims 1 to 5, characterized in that the antigen receptor is an immunoglobulin and the polypeptide chain is the heavy chain or the light chain of the immunoglobulin.

**7.** A method according to any of claims 1 to 5, characterized in that the antigen receptor is a T cell receptor and the polypeptide chain is the α, β, γ or δ chain of the T cell receptor.

**8.** A method according to claim 6, characterized in that the cells are B cell lymphoma cells.

**9.** A method according to claim 7, characterized in that the cells are T cell lymphoma cells.

**10.** A method according to any of claims 1 to 9, characterized in that the no amplification bias for certain sequences occurs.

**11.** A method for determining the immunoglobulin idiotype expressed by certain cells comprising
(a) providing DNA encoding at least the variable region of the heavy chain of the immunoglobulin and DNA encoding at least the variable region of the light chain of the immunoglobulin by a method according to claim 6;
(b) determining the nucleotide sequence of the heavy chain DNA and of the light chain DNA from (a).

**12.** A method for the preparation of an Fab fragment of an immunoglobulin expressed by certain cells comprising
(a) providing DNA encoding at least the variable region of the heavy chain of the immunoglobulin and DNA encoding at least the variable region of the light chain of the immunoglobulin by a method according to claim 6;
(b) cloning the heavy chain DNA and the light chain DNA into an expression vector;
(c) introducing the expression construct into a host cell;
(d) culturing the host cells under conditions such that the Ig heavy chain and Ig light chain are expressed; and optionally separating the Fab fragment.

**13.** A method according to claim 12, characterized in that the host cell is a bacterial cell.

**14.** A method according to claim 13, characterized in that the expression products are secreted into the periplasmic space of the bacterial cell.

**15.** A method according to any of claims 12 to 14, characterized in that the DNA is expressed in a dicistronic manner.

**16.** A method according to any of claims 12 to 15, characterized in that the Fab fragment does not contain any foreign amino acids within the immunoglobulin sequence.

**17.** Fab fragment prepared by a method according to any of claims 12 to 16.

**18.** Recombinantly produced Fab fragment according to claim 17 containing no foreign amino acids.

**19.** A method for determining which antigen is recognized by a lymphoma immunoglobulin comprising contacting an idiotypic Fab fragment according to claim 17 or 18 with a composition potentially containing the antigen; and optionally separating the Fab-antigen complex.

**20.** Vaccine composition containing an Fab fragment according to claim 17 or 18.

**21.** A method for the preparation of stimulated cytotoxic T cells against idiotype immunoglobulin of malignant lymphoma comprising
a) determining the idiotype expressed by the lymphoma cells by a method according to claim 11;
b) providing antigen presenting cells in vitro which present fragments of the idiotype on their surface;
c) contacting T cells in in vitro culture with the antigen presenting cells from b).

**26.** A method for the preparation of stimulated cytotoxic T cells against T cell receptors of malignant lymphoma comprising
a) determining the specific T cell receptor type expressed by the lymphoma cells by preparing DNA according to claim 7 and determining the nucleotide sequence of said DNA;
b) providing antigen presenting cells in vitro which present fragments of the T cell receptor on their surface;
c) contacting T cells in in vitro culture with the antigen presenting cells from b).

**23.** A method according to claim 21 or 22 characterized in that the T cells and the APC are autologous cells.

**24.** A method according to claim 21 or 22 characterized in that the T cells and the APC are allogeneic cells.

**25.** A method according to any of claims 21 to 24, characterized in that the APC are loaded with antigen by contacting the APC with idiotypic Fab fragments or T cell receptor fragments in vitro.

**26.** A method according to any of claims 21 to 24, characterized in that the APC are loaded with antigen by transferring DNA encoding at least part of the idiotype or of the T cell receptor into the APC such that the DNA sequences are expressed.

**27.** A stimulated T cell prepared by a method according to any of claims 21 to 26.

**28.** The use of anchored PCR for the preparation of V-region nucleotide sequences of immunoglobulins or T cell receptors.

**29.** The use of anchored PCR for determining the idiotype of immunoglobulins.

**30.** The use of anchored PCR for the preparation of Fab fragments.

**31.** The use of anchored PCR for the preparation of stimulated cytotoxic T cells.
